# EUROPEAN PATENT APPLICATION

(11) **EP 4 085 949 A1**
(43) Date of publication of application: **09.11.2022**
(21) Application number: 20909236.0
(22) Date of filing: 18.11.2020
(51) Int. Cl.: A61M 5/32, A61L 31/04, A61F 9/00, A61B 17/00

(54) **SELF-SEALABLE INJECTION NEEDLE FOR INHIBITING FORMATION OF FISTULA ON EYEBALL, AND METHOD FOR MANUFACTURING SAME**

(30) Priority: 31.12.2019 KR 20190179379; 21.04.2020 KR 20200047768
(71) Applicant: Innotherapy Inc., Seoul 07282 (KR)
(72) Inventor: LEE, Moon Sue, Seoul 06544 (KR); KOH, Mi Young, Incheon 21458 (KR); KIM, Soomi, Daejeon 34052 (KR); SONG, Jong Suk, Seoul 06798 (KR); EOM, Youngsub, Ansan-si Gyeonggi-do 15355 (KR)
(74) Representative: Mewburn Ellis LLP
(86) International application number: PCT/KR2020/016274
(87) International publication number: WO 2021/137430

(57) **Abstract**

The present invention relates to a self-sealable injection needle for inhibiting formation of fistula on an eyeball, and a method for manufacturing same. More specifically, the self-sealable injection needle for inhibiting formation of fistula on an eyeball, which is coated with hyaluronic acid on the surface thereof, can immediately close a hole that is inevitably formed when the injection needle is pulled out of an eyeball during the application of an ocular injection to prevent leakage of aqueous humor and to prevent backflow of drug out of the eyeball and can block bacterial penetration to prevent infection in the eyeball.

## Description

### TECHNICAL FIELD

The present invention relates to a self-sealing injection needle for inhibiting the formation of fistula on the eyeball and a method of preparing the same, and more particularly to a self-sealing injection needle for inhibiting the formation of fistula on the eyeball in which the surface of the injection needle is coated with hyaluronic acid, and a method of preparing the same.

### BACKGROUND ART

With increases in the average lifespan and in the elderly population, eyes are used for a longer time, and the incidence of glaucoma, diabetic retinopathy, and macular degeneration, which are chronic diseases affecting the blood vessels of the eye, commonly known as the three major diseases causative of blindness, is increasing.

The number of glaucoma patients increased by 49% over the past four years from 515,000 in 2011 to 767,000 in 2015. During the same period, the number of diabetic retinopathy patients increased by about 24%, and the number of macular degeneration patients increased by about 48%. As of 2015, the total number of patients suffering from the three major blindness-causing diseases has exceeded about 1.2 million. If this trend continues, the number of patients is expected to increase to 200 million worldwide by 2020.

The ophthalmic diseases described above are treated using intravitreal injection in which an antibody or anti-inflammatory agent is directly injected into the vitreous cavity.

Intravitreal injection is a technique widely used in drug therapy for various diseases afflicting the posterior segment of the eye. In particular, following intravitreal injection of triamcinolone acetonide to treat diabetic retinopathy, macular edema, and uveitis, recently, intravitreal injection of an anti-vascular endothelial growth factor (anti-VEGF) has been attempted in various ways to treat age-related macular degeneration and other diseases, leading to good results, so the popularity of eye injections has rapidly increased (Gupta O.P. et al., Ophthalmology. 2010 Nov;117(11):2134-40; Scanlon P.H. et al., Health Technol. Assess. 2015 Sep;19(74):1-116; Rasmussen A. et al., Acta. Ophthalmol. 2017 Mar;95(2):128-132).

Accordingly, interest in complications arising from intravitreal injection has increased. In particular, despite relatively low incidence compared to other complications, infectious endophthalmitis is a complication that may lead to critical results such as blindness, so attention must be paid thereto (Souied E.H. et al., A Retrospective Claims Database Analysis. Ophthalmic Epidemiol. 2016;23(2):71-9; Rayess N. et al., Br J. Ophthalmol. 2016 Aug;100(8):1058-61; VanderBeek B.L. et al., JAMA Ophthalmol. 2015 Oct; 133(10):1159-64). However, intravitreal injection requires repeated drug administration at temporal intervals, rather than a single injection, because drug efficacy does not last. As the frequency of administration increases, intraocular injection is repeated, so bacteria may enter the eyeball along the passage of the injection needle, increasing the risk of eye infection. When analyzing bacteria causative of intraocular infections due to ocular injection, it is known that most of them are *Staphylococcus aureus* residing on the surface of the eyeball, and also that the bacteria penetrate through the passage formed during and after ocular injection to thus cause intraocular infection.

Although intraocular infection is the most frightening complication in ophthalmology, which may lead to blindness, current methods capable of preventing such infectious endophthalmitis merely involve the use of a sterile environment, a povidone iodine solution, and a sterilized catheter. Therefore, there is an urgent need for a method capable of reliably preventing the occurrence of infectious endophthalmitis accompanying the use of ocular injections.

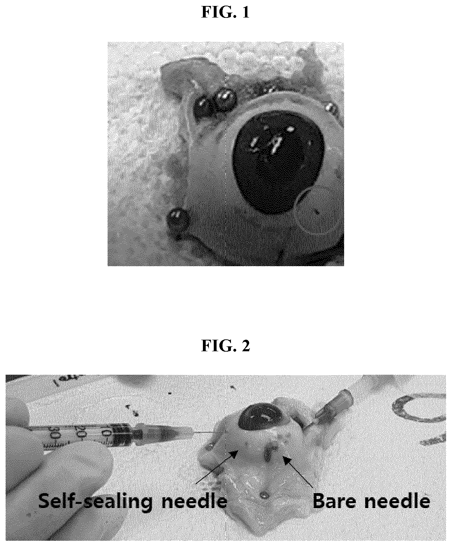

A hemostatic needle coated with crosslinked chitosan which is functionalized with a catechol group and oxidized catechol according to Korean Patent No. 10-1576503 awarded to the present inventors is capable of suppressing bleeding that occurs during injection. However, when this needle is used for ocular tissue, adhesion to the tissue is strong and friction with the tissue is high, so the user feels a sensation of stiffness, and the eyeball is observed to be pressed during injection. Furthermore, when the drug is repeatedly injected into the white part of the eyeball during clinical application, as shown in FIG. 1, chitosan having a catechol group introduced thereto remains in the injected white region, and the injection site takes on a distinctive color due to a change in the color of the polymer upon oxidation over time, which is undesirable.

Accordingly, the present inventors have made great efforts to solve the above problems, and ascertained that, when the surface of an injection needle is coated with a biocompatible polymer such as gelatin, collagen, hyaluronic acid, or the like, it is possible to prevent drug leakage by immediately closing the hole that is inevitably formed when the injection needle is removed from the eyeball after ocular injection, and to prevent intraocular infection by blocking bacterial penetration, thus culminating in the present invention.

### SUMMARY OF THE INVENTION

It is an object of the present invention to provide a self-sealing injection needle for inhibiting the formation of fistula on the eyeball after injection, thus preventing leakage of aqueous humor, drug leakage, and bacterial penetration, and a method of preparing the same.

In order to achieve the above object, the present invention provides an injection needle for inhibiting the formation of fistula on the eyeball, in which the surface of the injection needle is coated with at least one biocompatible polymer selected from the group consisting of hyaluronic acid and collagen.

In addition, the present invention provides a method of preparing the injection needle for inhibiting the formation of fistula on the eyeball including (a) subjecting an injection needle to oxygen plasma treatment and (b) coating the injection needle with a solution of at least one biocompatible polymer selected from the group consisting of hyaluronic acid and collagen.

### BRIEF DESCRIPTION OF THE DRAWINGS

FIG. 1 shows a photograph in which an injection needle coated with chitosan having a catechol group introduced thereto according to an embodiment of the present invention is applied to ocular tissue;
FIG. 2 shows a photograph comparing the sealing capability of a self-sealing injection needle coated with hyaluronic acid according to an embodiment of the present invention depending on the molecular weight of hyaluronic acid, with the sealing capability of a general injection needle;
FIG. 3 shows an image of a 30G injection needle coated with hyaluronic acid in two steps according to an embodiment of the present invention;
FIG. 4 shows graphs of the results of analysis of the self-sealing injection needle coated with hyaluronic acid according to an embodiment of the present invention using a needle penetration force tester;
FIG. 5 shows graphs of results confirming the concentration values of six cytokines in the aqueous humor of a rabbit eyeball injected with the self-sealing injection needle coated with hyaluronic acid according to an embodiment of the present invention; and
FIG. 6 shows graphs of results confirming the concentration values of six cytokines in the vitreous fluid of a rabbit eyeball injected with the self-sealing injection needle coated with hyaluronic acid according to an embodiment of the present invention.

### DETAILED DESCRIPTION OF THE INVENTION

Unless otherwise defined, all technical and scientific terms used herein have the same meanings as those typically understood by those skilled in the art to which the present invention belongs. In general, the nomenclature used herein is well known in the art and is typical.

In the present invention, it has been confirmed that, when an injection needle is manufactured by coating the surface of the injection needle with hyaluronic acid, it is capable of immediately closing the hole that is inevitably formed when the injection needle is removed from the eyeball after ocular injection, thereby preventing leakage of aqueous humor, extraocular expulsion of the drug, and bacterial penetration, ultimately preventing intraocular infection.

Accordingly, an aspect of the present invention pertains to an injection needle for inhibiting the formation of fistula on the eyeball, in which the surface of the injection needle is coated with at least one biocompatible polymer selected from the group consisting of hyaluronic acid and collagen.

Another aspect of the present invention pertains to a method of preparing the injection needle for inhibiting the formation of fistula on the eyeball including (a) subjecting an injection needle to plasma treatment and (b) coating the injection needle with a solution of at least one biocompatible polymer selected from the group consisting of hyaluronic acid and collagen.

Hereinafter, a detailed description will be given of an injection needle for inhibiting the formation of fistula on the eyeball according to the present invention and a method of preparing the same.

The injection needle for inhibiting the formation of fistula on the eyeball according to the present invention is characterized in that the surface of the injection needle is coated with at least one biocompatible polymer selected from the group consisting of hyaluronic acid and collagen.

The biocompatible polymer applied on the injection needle for inhibiting the formation of fistula on the eyeball according to the present invention may be an anionic polymer, a cationic polymer, a neutral polymer, or a synthetic polymer. Preferably, it is an anionic polymer such as hyaluronic acid, alginate, carboxymethylcellulose, etc., a cationic polymer such as chitosan, etc., a neutral polymer such as gelatin, collagen, dextran, etc., or a synthetic polymer such as polyethylene glycol, etc. In particular, the biocompatible polymer is preferably hyaluronic acid, collagen, or a mixture of hyaluronic acid and collagen.

The ocular injection needle coated with hyaluronic acid according to the present invention has good biocompatibility with ocular tissue, and has a sealing effect in order to suppress not only drug leakage but also intraocular inflammation due to entry of bacteria that may exist in the eyelids into the injection passage. Here, there is an advantage in that the sensation of use thereof is not stiff compared to a general syringe because frictional force with the tissue during injection is reduced

In the present invention, the molecular weight of hyaluronic acid may be 20 to 1000 KDa, preferably 66 to 850 KDa, and more preferably 151 to 749 KDa.

If the molecular weight of hyaluronic acid is less than 20 KDa, the extent of water absorption is low due to the low molecular weight thereof, so the expansion rate is low, and also, it is easily absorbed into the tissue owing to weak physical properties, making it impossible to sufficiently block the fistula. On the other hand, if it exceeds 1000 KDa, hard physical properties may result due to the high molecular weight thereof, so the sensation of use may be stiff upon injection, or the polymer coating film applied on the injection needle may not be fixed to the tissue but may peel off and may be pushed from the fistula created after injection, making it impossible to block the fistula.

In the present invention, the amount of hyaluronic acid that is applied may be 12.6 to 276 µg, preferably 12.6 to 138 µg.

If the amount of hyaluronic acid is less than 12.6 µg, the amount of the polymer is small, so there is little swelling due to moisture in the tissue, and the fistula cannot be sufficiently blocked due to the low physical properties. On the other hand, if it exceeds 276 µg, the polymer coating film may become thick, so the sensation of use may be stiff, and the eyeball may be pressed by force during injection.

In the present invention, the coating thickness of the injection needle may be 0.1 to 40 µm, and preferably, the coating thickness of the front portion of the needle, which touches the eyeball first, is 0.1 to 10 µm, and the coating thickness of the rear portion thereof is 20 to 40 µm. Here, if the coating thickness exceeds 40 µm, the polymer coating film may become thick, and the sensation of use may be stiff, and the eyeball may be pressed by force during injection.

In the present invention, the eyeball may be the eyeball of a subject suffering from an ophthalmic disease selected from the group consisting of diabetic retinopathy, macular degeneration, uveitis, retinopathy of prematurity, corneal graft rejection, neovascular glaucoma, and proliferative retinopathy, and preferably diabetic retinopathy, macular degeneration, or uveitis. Any ophthalmic disease may be the target of treatment, so long as it is treatable through ocular injection.

A method of preparing the injection needle for inhibiting the formation of fistula on the eyeball according to the present invention may include (a) subjecting an injection needle to plasma treatment and (b) coating the injection needle with a solution of at least one biocompatible polymer selected from the group consisting of hyaluronic acid and collagen.

In the present invention, the coating in step (b) may be performed at a temperature of 15 to 25°C for 0.5 to 18 hours.

In the present invention, oxygen plasma treatment may be performed at a temperature of 15 to 25°C for 5 minutes to 20 minutes, preferably 8 minutes to 12 minutes, and most preferably 10 minutes.

In the present invention, the molecular weight of the hyaluronic acid may be 20 to 1000 KDa, and the concentration of the solution of hyaluronic acid may be 0.1 to 2.0 wt%.

The present invention also provides a method of immediately closing the hole created after surgery by applying the self-sealing injection needle for inhibiting the formation of fistula on the eyeball to the hole remaining after removal of the instrument in the case of surgery performed by forming a hole in the eyeball and inserting an instrument into the eyeball, as in pars plana vitrectomy.

A better understanding of the present invention may be obtained through the following examples. These examples are merely set forth to illustrate the present invention, and are not to be construed as limiting the scope of the present invention, as will be apparent to those skilled in the art.

### [Examples]

### Example 1: Selection of polymer suitable for ocular tissue

Ocular syringes respectively coated with hyaluronic acid (HA), alginate, carboxymethylcellulose (CMC), chitosan, gelatin, collagen, dextran, and chitosan having a catechol group introduced thereto as a polymer were manufactured.

The chitosan having a catechol group introduced thereto was a polymer disclosed in a prior patent (Korean Patent No. 10-1576503, title of invention: Hemostatic needle coated with crosslinked chitosan which is functionalized with catechol group and oxidized catechol).

The sealing capability and the sensation of use in pig ocular tissue were compared and confirmed using the ocular syringes coated with various biocompatible polymers, and five independent trials were performed.

Table 1 below shows the results of comparison of sealing capability and sensation of use of ocular syringes coated with various biocompatible polymers. It was confirmed that the ocular syringe coated with hyaluronic acid exhibited superior sealing capability and sensation of use compared to when other biocompatible polymers were used. Here, the sealing capability was determined based on leakage of the solution after injection into the pig's eyeball, and the sensation of use was judged by the sensation experienced by the user when the polymer coating of the injection needle punctures the eyeball during ocular injection and the eyeball-pressing phenomenon. A high score was awarded when the sensation of use was good.

As such, chitosan having a catechol group introduced thereto is capable of suppressing bleeding during injection, but when it was used for ocular tissue, the user felt a sensation of stiffness due to strong adhesion thereof to the tissue and high friction with the tissue, and eyeball pressing was observed upon injection. In addition, when the drug is repeatedly injected into the white part of the eyeball during clinical application, there is the possibility of discoloration of the injection site upon oxidation over time because chitosan having a catechol group introduced thereto is left behind in the injected white region, as shown in FIG. 1.

However, the ocular syringe coated with hyaluronic acid has good biocompatibility with the ocular tissue, and has a sealing effect in order to suppress not only drug leakage but also intraocular inflammation due to entry of bacteria that may exist in the eyelids into the injection passage. As such, hyaluronic acid and collagen were selected as polymers suitable for ocular injection because the sensation of use was not stiff compared to a general syringe due to reduction in frictional force with the tissue upon injection.

**[Table 1]**

| Classification | Extent of sealing (%) | Sensation of use (0-5 scores) |
|---|---|---|
| Hyaluronic acid | 80 | 4 |
| Alginate | 80 | 1 |
| Carboxymethyl cellulose | 0 | 1 |
| Gelatin | 80 | 1 |
| Collagen | 100 | 2 |
| Dextran | 40 | 4 |
| Chitosan having catechol group introduced thereto | 100 | 0 |
| General injection needle having no coating | 0 | 5 |

### Example 2: Manufacture of self-sealing injection needle

In order to manufacture a self-sealing injection needle, 15 mg of hyaluronic acid (HA) having a molecular weight ranging from 10 to 1190 KDa was dissolved in 1 mL of distilled water.

A general injection needle (30G) was subjected to oxygen plasma treatment for 10 minutes, a hyaluronic acid solution having a molecular weight ranging from 10 to 1190 KDa was applied on the injection needle, and the injection needle was coated therewith while rotating at room temperature for 1 hour. The above procedure was repeated twice to afford a self-sealing injection needle.

### [Experimental Examples]

### Experimental Example 1: Comparison of sealing performance in pig ocular tissue depending on molecular weight of hyaluronic acid

In this experimental example, in order to compare and evaluate the sealing effects of self-sealing injection needles (30G) coated with hyaluronic acid having various molecular weights, as in Example 2, when the dye solution was injected into the pig ocular tissue and then the injection needle was removed therefrom, leakage of the dye solution was observed.

FIG. 2 and Table 2 below show the results of comparison of sealing capability depending on the molecular weight of hyaluronic acid. The general injection needle (30G) had no sealing capability due to leakage of the dye solution through the injection passage, whereas the self-sealing injection needle coated with hyaluronic acid having a molecular weight of 176-350 KDa and the self-sealing injection needle coated with hyaluronic acid having a molecular weight of 601-850 KDa exhibited the best sealing effect in the ocular tissue.

**[Table 2]**

| Classification | Extent of sealing (%) |
|---|---|
| 30G general needle | 0 |
| Needle coated with HA having 10-20 KDa | 0 |
| Needle coated with HA having 176-350 KDa | 66.7 |
| Needle coated with HA having 601-850 KDa | 83.3 |
| Needle coated with HA having 1001-1190 KDa | 14.3 |

### Experimental Example 2: Control and confirmation of thickness of hyaluronic acid coating on injection needle

In this experimental example, an injection needle was manufactured by adjusting the thickness of the hyaluronic acid coating film on the injection needle in a manner in which the front portion and the rear portion of the injection needle were coated at different thicknesses with hyaluronic acid having a molecular weight of 601-850 KDa, which had the best sealing effect in the ocular tissue in Experimental Example 1, so as to exhibit a sealing effect and simultaneously enable injection without stiffening by reducing friction with the tissue during use.

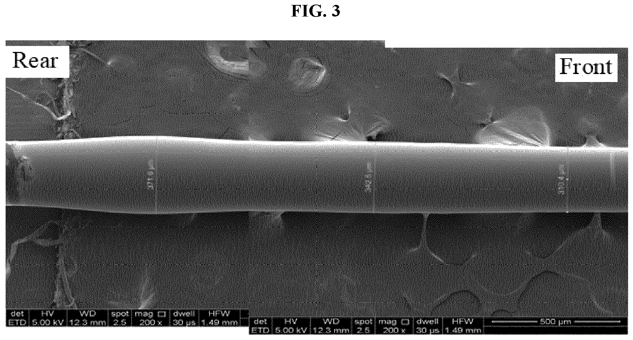

From an enlarged view of the injection needle, the front portion and the rear portion of which were coated with hyaluronic acid at different thicknesses, using a scanning electron microscope (SEM), the shape of the coating film on the injection needle was observed. FIG. 3 shows an image of a 30G injection needle coated with hyaluronic acid, in which the front portion of the injection needle coated with hyaluronic acid was measured to be about 310.4 µm thick, and the rear portion thereof was measured to be 371.6 µm thick.

The 30G injection needle had the following specifications.

**[Table 3]**

| Gauge | Outer diameter (mm) | Inner diameter (mm) |
|---|---|---|
| 30G | 0.30 | 0.15 |

It was confirmed that the front portion of the injection needle to be used for injection was coated thinly in order to reduce friction with the tissue, and the rear portion thereof was coated to be thicker than the front portion in order to exhibit sealing performance.

### Experimental Example 2-1: Confirmation of penetration force of self-sealing injection needle

In this experimental example, in order to evaluate the ability of the hyaluronic acid coating film on the manufactured self-sealing injection needle (30G) to penetrate tissue when injected into biotissue, analysis was performed using a needle penetration force tester. Three independent trials were performed.

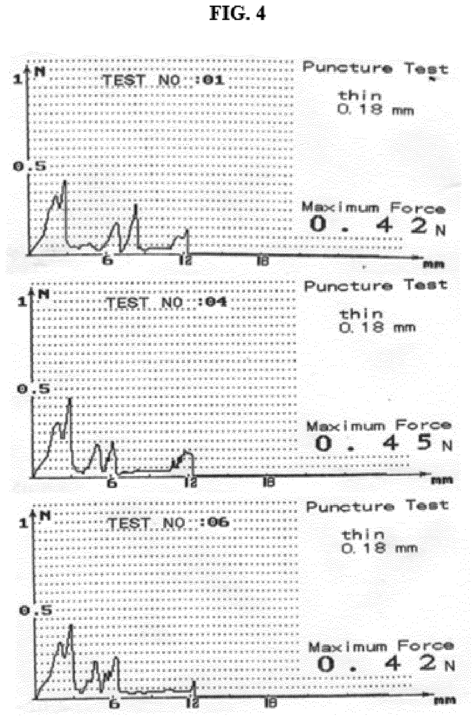

FIG. 4 shows the results of analysis using the needle penetration force tester. When the needle penetrates artificial tissue, the greatest force was measured to be 0.42 to 0.45 N, and penetration force at the position where the hyaluronic acid coating film was applied was measured to be 0.20 to 0.30 N, which was smaller than the greatest force.

### Experimental Example 2-2: Comparative evaluation of sealing performance and sensation of use in pig ocular tissue

In this experimental example, in order to compare and evaluate the sealing performance and sensation of use of an injection needle, the front portion and the rear portion of which were coated with hyaluronic acid at different thicknesses, and an injection needle, the front portion and the rear portion of which were coated with hyaluronic acid at the same thickness, when the dye solution was injected into pig ocular tissue and then the injection needle was removed therefrom, whether the dye solution leaked was observed, and at the same time, the sensation experienced by the user when the HA coating punctures the ocular tissue during injection and the eyeball-pressing phenomenon were compared and evaluated.

Table 4 below shows the results of comparison of sealing performance and sensation of use depending on the coating thickness of the front portion and the rear portion of the injection needle. Compared with the injection needle, the front portion and the rear portion of which were coated with hyaluronic acid at the same thickness, it was confirmed that the injection needle, the front portion and the rear portion of which were coated with hyaluronic acid at different thicknesses, maintained the sealing effect and had an improved sensation of use.

**[Table 4]**

| Classification | Extent of sealing (%) | Sensation of use (0-5 scores) |
|---|---|---|
| Injection needle coated with HA at different front portion/rear portion thicknesses | 80.0 | 4 |
| Injection needle coated with HA at the same front portion/rear portion thickness | 83.3 | 2 |
| General injection needle having no coating | 0 | 5 |

### Experimental Example 2-3: Confirmation of sealing performance in rabbit ocular injection model

In this experimental example, sealing performance was compared and evaluated by applying the manufactured self-sealing injection needle (30G) and a general injection needle (30G) to a rabbit ocular injection model. The animal used for evaluation was a New Zealand white rabbit. The test groups were divided into a 'control group' to which the general injection needle was applied and an 'experimental group' to which the self-sealing injection needle manufactured in Example 2 was applied. Three independent trials were performed.

After the rabbit was completely anesthetized, the rabbit eyeball was injected with the dye solution using the general injection needle or the self-sealing injection needle, and then leakage of the dye solution through the injection passage was visually observed.

Table 5 below shows the results of visually observing leakage of the injected dye solution through the injection passage after injecting the dye solution using the general injection needle or the self-sealing injection needle. It was confirmed that the dye solution leaked through the injection passage when using the general injection needle but did not leak when using the self-sealing injection needle.

**[Table 5]**

| Classification | Sealing | Extent of sealing (%) |
|---|---|---|
| General injection needle | x | 0 |
| | x | |
| | x | |
| Self-sealing injection needle | o | 100 |
| | o | |
| | o | |

### Experimental Example 2-4: Identification of inflammatory cytokines in aqueous humor and vitreous humor in eyeball after rabbit ocular injection

In this experimental example, the following experiment was performed in order to evaluate whether an inflammatory response was caused by the coating material after ocular injection using the manufactured self-sealing injection needle (30G) compared to a general injection needle (30G).

Specifically, the rabbit was completely anesthetized and then subjected to ocular injection four times. The above procedure was performed in the same manner on days 3 and 7. After completion of ocular injection on day 7, the eyeball of the animal was extracted and stored frozen, after which aqueous humor and vitreous fluid in the eyeball were separated from each other. The concentrations of six inflammation-related cytokines, namely IL-4, IL-17, IL-1b, PEG2, TNG-α, and IFN-g, in each of the aqueous humor and the vitreous fluid were analyzed through ELISA.

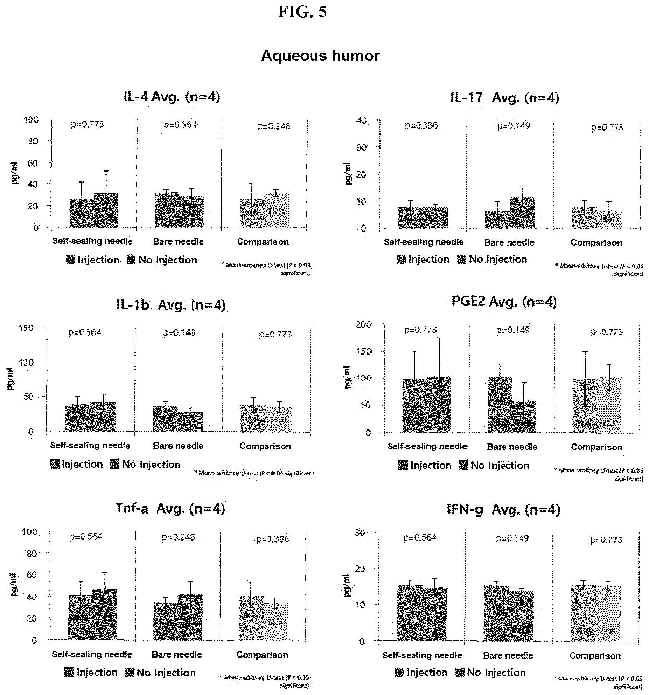

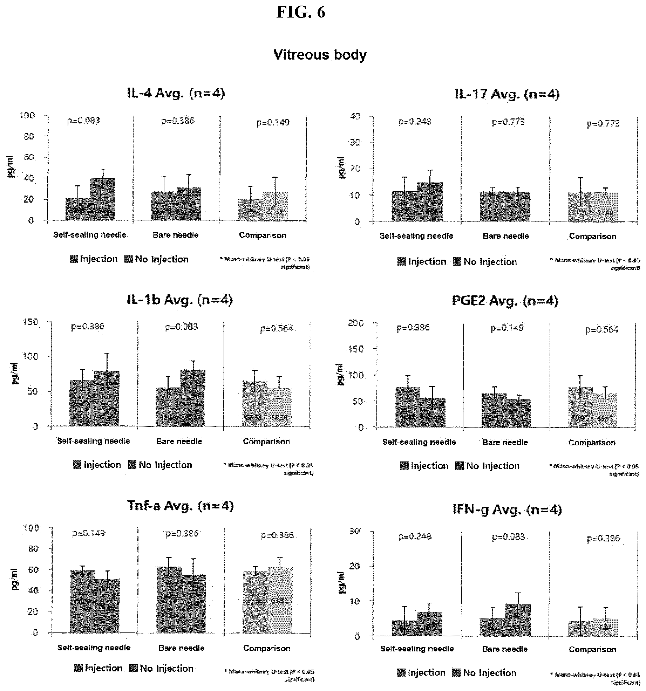

FIG. 5 shows results confirming the concentration values of six cytokines in the aqueous humor, and FIG. 6 shows results confirming the concentration values of six cytokines in the vitreous fluid. Here, it was confirmed that there was no difference in the inflammatory response when the inflammatory cytokine concentration values were compared upon ocular injection using the general injection needle and the self-sealing injection needle.

### INDUSTRIAL APPLICABILITY

According to the present invention, an injection needle coated with hyaluronic acid for inhibiting the formation of fistula on the eyeball is configured such that the surface of an injection needle for use in the eyeball is coated very thinly with an ocular self-sealing material, thus immediately closing the hole that is inevitably formed when the injection needle is removed from the eyeball after ocular injection, thereby preventing leakage of aqueous humor and bacterial penetration, ultimately preventing intraocular infection.

Moreover, it is possible to prevent extraocular expulsion of drugs, which often occurs upon injection of expensive drugs such as Lucentis or Eylea, used for various eye diseases such as macular degeneration and the like (costing, for example, about 1 million won per injection of Lucentis), so the required dose set by the pharmaceutical company can be reliably administered, thereby greatly reducing the burden on a patient.

Although specific embodiments of the present invention have been disclosed in detail above, it will be obvious to those of ordinary skill in the art that the description is merely of preferable exemplary embodiments and is not to be construed as limiting the scope of the present invention. Therefore, the substantial scope of the present invention is to be defined by the appended claims and equivalents thereof.

## Claims

1. An injection needle for inhibiting formation of fistula on an eyeball, in which a surface of the injection needle is coated with at least one biocompatible polymer selected from the group consisting of hyaluronic acid and collagen.

2. The injection needle of claim 1, wherein the biocompatible polymer is hyaluronic acid.

3. The injection needle of claim 1, wherein the biocompatible polymer is collagen.

4. The injection needle of claim 1, wherein the biocompatible polymer is a mixture of hyaluronic acid and collagen.

5. The injection needle of claim 2, wherein a molecular weight of the hyaluronic acid is 20 to 1000 KDa.

6. The injection needle of claim 1, wherein a coating thickness of the injection needle is 0.1-40 µm.

7. The injection needle of claim 1, wherein a coating thickness of a front portion of the injection needle is 0.1 to 10 µm, and a coating thickness of a rear portion thereof is 20 to 40 µm.

8. The injection needle of claim 1, wherein the eyeball is an eyeball of a subject suffering from an ophthalmic disease selected from the group consisting of diabetic retinopathy, macular degeneration, uveitis, retinopathy of prematurity, corneal graft rejection, neovascular glaucoma, and proliferative retinopathy.

9. A method of preparing the injection needle of claim 1, comprising:
(a) subjecting an injection needle to plasma treatment; and
(b) coating the injection needle with a solution of at least one biocompatible polymer selected from the group consisting of hyaluronic acid and collagen.

10. The method of preparing the injection needle of claim 9, wherein the biocompatible polymer is hyaluronic acid.

11. The method of preparing the injection needle of claim 9, wherein the biocompatible polymer is collagen.

12. The method of preparing the injection needle of claim 9, wherein the biocompatible polymer is a mixture of hyaluronic acid and collagen.

13. The method of preparing the injection needle of claim 9, wherein the coating in step (b) is performed at a temperature of 15 to 25°C for 0.5 to 18 hours.

14. The method of preparing the injection needle of claim 9, wherein the plasma treatment is performed at a temperature of 15 to 25°C for 5-20 minutes.

15. The method of preparing the injection needle of claim 10, wherein a molecular weight of the hyaluronic acid is 20 to 1000 KDa.

16. The method of preparing the injection needle of claim 10, wherein a concentration of a solution of the hyaluronic acid is 0.1 to 2.0 wt%.
